# EUROPEAN PATENT APPLICATION

(11) **EP 4 768 021 A1**
(43) Date of publication of application: **01.07.2026**
(21) Application number: 25210020.1
(22) Date of filing: 21.10.2025
(51) Int. Cl.: A61K 9/20, A61K 31/44, A61K 47/36, A61P 25/00

(54) **EFFICIENT PRODUCTION PROCESS FOR SUSTAINED-RELEASE FAMPRIDINE TABLETS**

(30) Priority: 25.12.2024 TR 2024020494
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); IPEK, Celaleddin, Istanbul (TR); PEHLIVAN AKALIN, Nur, Istanbul (TR); ULUSOY BOZYEL, Muge, Istanbul (TR); YENI, Songul, Istanbul (TR); DAGCIOGLU, Erdal, Istanbul (TR)
(74) Representative: Genç Ilhan, Oznur

(57) **Abstract**

The present invention relates to a process for sustained-release fampridine tablets characterized by their high homogeneity and flowability.

## Description

### Field of the Invention

The present invention relates to a process for sustained-release fampridine tablets characterized by their high homogeneity and flowability.

### Background of the Invention

Multiple sclerosis (MS) is a progressive neurological disease that damages the central nervous system by targeting the myelin sheath. The demyelination of nerve fibers disrupts electrical signals, causing delayed or blocked nerve transmission and resulting in disabling symptoms. Effective treatments to improve nerve signal conduction are currently limited.

Potassium channel blockers are a group of compounds known to enhance the conduction of nerve impulses, making them a key area of interest in the symptomatic management of conditions such as spinal cord injury, MS, and Alzheimer's disease. Among these, aminopyridines, a subclass of potassium channel blockers, have shown particular promise in treating neurological disorders. One example, 4-aminopyridine (4-AP), also called fampridine, works by inhibiting potassium flow during nerve impulse transmission, effectively aiding in restoring conduction in damaged or demyelinated nerves.

Fampridine is a potassium channel blocker indicated for symptomatic improvement of walking in adults with multiple sclerosis, including relapsing remitting, secondary progressive, progressive relapsing and primary progressive. The chemical name of fampridine is 1,4-dihydropyridin-4-imine and its chemical structure is shown in Formula I.

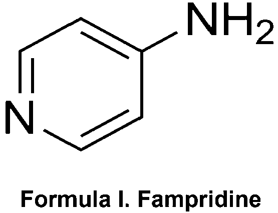

Fampridine is available under the trade name Ampyra from Acorda Therapeutics is available as 10-mg film-coated, sustained-release, white to off-white, oval tablets.

Fampridine has been formulated in sustained-release matrix tablets at various concentrations ranging from 5 to 40 mg.

There is no known cure for MS until now. Current treatments typically focus on accelerating patient's recovery from attacks, slowing the progression of the disease and restraining MS symptoms.

Research in patients with multiple sclerosis (MS), including phase 1, 2 and 3 clinical trials, has shown that the drug fampridine can improve many types of nerve function damaged by the disease, with particular attention paid to its effects on improving walking and leg strength.

WO2017058869A1 discloses sustained release fampridine tablets, which include a core and a coating. The core comprises fampridine, a polyethylene oxide with a molecular weight between about 1,000,000 and 10,000,000, and a mixture comprising polyvinyl acetate and polyvinyl pyrrolidone; and a coating comprises ethylcellulose surrounding said compressed core.

US20170071923A1 provides a sustained release oral dosage form containing fampridine that can be administered once daily. The dosage form includes fampridine as the active pharmaceutical ingredient and the excipients comprising osmotic agents in a tablet core.

EP1732548B2 discloses the wet granulation method as an option for the production of sustained-release fampridine tablets; however, it does not provide detailed information regarding the steps involved in this production method and technical effects of this method.

In addition, this document specifically identifies microcrystalline cellulose, sold under the name Avicel PH 101, as the particularly preferred diluent for sustained-release fampridine tablets. However, it provides no information regarding other types of microcrystalline cellulose or their technical effects in this prior art document.

The prior art encompasses various documents detailing production processes for sustained-release fampridine tablets. However, these processes are notably limited by issues of poor flowability and/or homogeneity. To date, no existing solutions or recommendations have effectively resolved these challenges, either individually or simultaneously.

### Detailed description of the Invention

The main object of the present invention is to present a process of the sustained-release tablet comprising fampridine having improved flow properties. Improved flow properties of the tablet provide high-speed tableting, reducing flow-related issues such as weight variation or inconsistent die filling.

Another object of the present invention is to improve homogeneity of the sustained-release tablets comprising fampridine.

The sustained-release tablet comprising fampridine comprises the steps below provided the desired fluidity and also helped to ensure homogeneity with the solvents used. In this way, the desired dissolution profile was achieved.

The invention relates to a process for sustained-release tablet comprising fampridine or salt thereof. The process comprises the following steps:
- granulating a mixture containing fampridine or a salt thereof, a diluent, a glidant with alcohol or water or alcohol/water mixture and
- further mixing with at least one rate-controlling polymer and with lubricant and forming the obtained mixture into a tablet.

The process further comprises the step of coating having one or more coating agents.

According to another embodiment of the present invention, alcohol or water or a mixtures thereof is used as solvent at the process. These solvents help to ensure high homogeneity without damaging the stability of fampridine.

Targeting of drugs to the colon following oral administration has also been accomplished by using rate-controlling polymers; also they act as binders that swell when hydrated by gastric media and delay absorption. In this invention, we have used cellulose derived polymers as rate-controlling polymers.

Cellulose and its derivatives have demonstrated to be versatile materials with unique chemical structure which provides a good platform for the construction of hydrogel networks with distinctive properties as respects of swelling ability and sensibility to external stimuli. The cellulosic rate-controlling polymer provides the desired dispersion of fampridine throughout the matrix and it imparts chemical and physical stability to the composition while providing sustained-release profile. This improved dosage stability is particularly important in compositions of the present invention containing low amounts of fampridine. By utilising a cellulosic rate-controlling polymer, stability is achieved while maintaining the desired sustained-release profile.

Suitable rate-controlling polymer is selected from the group comprising ethylcellulose, methylcellulose, hydroxypropyl cellulose, hydroxypropyl methylcellulose (HPMC), hydroxyethyl cellulose, hydroxymethyl cellulose, sodium carboxymethylcellulose, carboxymethyl cellulose, nitrocellulose, methylcellulose or mixtures thereof.

According to another embodiment of the present invention, the rate-controlling polymer is hydroxypropyl methyl cellulose. This excipient provides stability while ensuring the desired sustained-release profile. Especially, a low-viscosity type of HPMC is used, for example; HPMC K100LV.

According to another embodiment of the present invention, the amount of hydroxypropyl methyl cellulose is between 25.0% to 70.0% by weight of the total composition. Preferably, it is between 42.0% to 65.0% by weight of the total composition. More preferably, it is between 52.0% to 65.0% by weight of the total composition.

Since low amounts of fampridine are used in the formulation, it is important to ensure content uniformity. When used in the particle sizes specified below, fampridine helped provide the desired dissolution profile by ensuring content uniformity.

As used here in, 'particle size' means the cumulative volume size distribution as tested by any conventionally accepted method such as the laser diffraction method (i.e. Malvern analysis). The term d (0.9) means the size at which %90 by volume of the particles are finer, the term d (0.5) means the size at which %50 by volume of the particles are finer, the term d (0.1) means the size at which %10 by volume of the particles are finer.

According to another embodiment of the present invention, a sustained-release tablet comprises fampridine and at least one pharmaceutically acceptable excipient wherein a d (0.9) particle size of fampridine is smaller than 120 µm and at least one cellulosic rate-controlling polymer.

According to one embodiment, fampridine has a d (0.9) particle size between 120 µm and 20 µm or between 100 µm and 25 µm or between 85 µm and 30 µm or between 80 µm and 35 µm. Use of particle size in this range ensures content uniformity.

According to another embodiment of the present invention, the amount of fampridine is between 0.1% to 7.0% by weight of the total composition. Preferably, it is between 1.2% to 3.5% by weight of the total composition.

Suitable diluents are selected from the group comprising microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to another embodiment of the present invention, the diluent is microcrystalline cellulose.

In a preferred embodiment according to the present invention, the diluent used is microcrystalline cellulose, identified by the code names PH-302 with an average particle size (D50) ranging from 70 to 140 µm and a loose density of 0.35 to 0.46 g/ml.

Compared to PH-101 microcrystalline cellulose, PH-302 microcrystalline cellulose exhibits higher average particle size and bulk density.

The use of PH-302 microcrystalline cellulose as a diluent in the tablet enhances the flow properties of the sustained-release tablets containing fampridine and prevents its hydrolysis or degradation caused by exposure to high-moisture-content excipients. Enhancing flow properties facilitates high-speed tableting and minimizes flow-related issues, such as weight variation or inconsistent die filling.

The synergistic combination of PH-302 microcrystalline cellulose in the wet granulation process for producing sustained-release fampridine tablets not only enhances homogeneity but also provides high flowability for the tablet formulation.

According to another embodiment of the present invention, the amount of microcrystalline cellulose is between 25.0% to 60.0% by weight of the total composition. Preferably, it is between 30.0% to 45.0% by weight of the total composition.

Suitable lubricants are selected from the group comprising from magnesium stearate, calcium stearate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, sodium stearyl fumarate, sodium lauryl sulphate or mixtures thereof.

According to another embodiment of the present invention, the lubricant is magnesium stearate.

According to another embodiment of the present invention, the amount of magnesium stearate is between 0.05 to 3.0 by weight of the total composition.

Suitable glidants are selected from the group comprising anhydrous colloidal silicon dioxide, corn starch, talc or mixtures thereof.

According to another embodiment of the present invention, the glidant is anhydrous colloidal silicon dioxide.

According to another embodiment of the present invention, the amount of anhydrous colloidal silicon dioxide is between 0.05 to 3.0 by weight of the total composition. Preferably, it is between 0.1% to 1.5% by weight of the total composition.

Suitable coating agents are selected from the group comprising hydroxypropyl methyl cellulose, polymethacrylates, polyalkylacrylates copolymers, hydroxyl propyl methyl cellulose, lactose monohydrate, hydroxypropyl cellulose, polyvinyl alcohol, polyethylene glycol, talc, polyvinyl alcohol-polyethylene glycol copolymers (Kollicoat^{®} IR), ethylcellulose dispersions (Surelease^{®}), polyvinylpyrrolidone, polyvinylpyrrolidone-vinyl acetate copolymer (PVP-VA), all kinds of Opadry^{®}, pigments, dyes, titanium dioxide, iron oxide or mixtures thereof.

According to another embodiment of the present invention, the coating agent is hydroxypropyl methyl cellulose, titanium dioxide, polyethylene glycol or mixtures thereof.

According to another embodiment of the present invention, the sustained-release composition comprises;
- Fampridine
- HPMC K100LV
- Microcrystalline cellulose PH302
- Anhydrous colloidal silicon
- Magnesium stearate

In the invention, the sustained-release tablet is obtained by wet granulation process.

When compared to other methods, such as dry granulation, the wet granulation process improves the homogeneity of sustained-release fampridine tablets.

According to one embodiment, the sustained-release tablet has a hardness of between 180 N and 300 N. This provides high chemical and mechanical stability; thus it is not brittle easily. Hardness test is done with Erweka Tablet Hardness Tester.

### Example 1:

| **Ingredients** | **Unit Amount (mg)** | **Amount (%)** | **Amount (%)** |
|---|---|---|---|
| Fampridine | 10 | 0.1 - 7.0 | 2.5 |
| HPMC K100LV | 240 | 25.0 - 70.0 | 60.0 |
| Microcrystalline cellulose PH302 | 147.6 | 25.0 - 60.0 | 36.9 |
| Anhydrous colloidal silicon | 1.60 | 0.05 - 3.0 | 0.4 |
| Magnesium stearate | 0.80 | 0.05 - 3.0 | 0.2 |
| **Total Core Tablet Weight** | **400** | **100.00** | **100.00** |
| OPADRY WHITE Y-1-7000 | | | |
| *Hydroxypropyl methyl cellulose 5 cP (Methocel E5 LV) %62,5* | 10.00 | | |
| *Titanium dioxide %31,25* | | | |
| *Polyethylene glycol 400 %6,25* | | | |
| **Total Coating Tablet Weight** | **410** | | |

According to another embodiment of the present invention, the wet granulation process comprises following production steps:
a) Weighing the fampridine and all excipients,
b) Mixing fampridine and glidant homogeneously,
c) Adding diluent into the mixture and mixing homogeneously,
d) Sieving the mixture,
e) Granulation with alcohol or water or alcohol/water mixture and followed by drying the granules and sieving them,
f) Adding rate-controlling polymer as the external phase and mixing,
g) Sieving lubricant and adding into the mixture and mixing,
h) Pressing the prepared mixture into tablets to ensure the tablet weight meets the specified requirements.

According to another embodiment of the present invention, the wet granulation process comprises following production steps:
a) Weighing the fampridine and all excipients,
b) Mixing fampridine and anhydrous colloidal silicon dioxide homogeneously,
c) Adding microcrystalline cellulose PH302 into the mixture and mixing homogeneously,
d) Sieving the mixture through a 0.6 mm round sieve,
e) Granulation with alcohol or water or alcohol/water mixture (70%) and followed by drying the granules at 50°C and sieving them through a 0.6 mm sieve.
f) Adding hydroxypropyl methylcellulose K100LV as the external phase and mixing for 5 min.
g) Sieving magnesium stearate and adding into the mixture and mixing for 3 min.
h) Pressing the prepared mixture into tablets to ensure the tablet weight meets the specified requirements.
i) Coating the tablets until the desired film-coated tablet weight is achieved.

## Claims

1. A process for producing a sustained-release tablet comprising fampridine or salt thereof, the process comprising the steps of:
- granulating a mixture containing fampridine or a salt thereof, a diluent, a glidant with alcohol or water or alcohol/water mixture and
- further mixing with at least one rate-controlling polymer and with lubricant and forming the obtained mixture into a tablet.

2. The process according to claim 1, further comprising the step of coating with one or more coating agents.

3. The process according to any of the previous claims, wherein fampridine has a d(0.9) particle size between 120 µm and 20 µm or between 100 µm and 25 µm or between 85 µm and 30 µm or between 80 µm and 35 µm.

4. The process according to any of the previous claims, wherein the amount of fampridine is between 0.1% to 7.0% by weight of the total composition.

5. The process according to any of the previous claims, wherein the amount of fampridine is between 1.2% to 3.5% by weight of the total composition.

6. The process according to any one of the previous claims, wherein rate-controlling polymer is selected from the group comprising ethylcellulose, methylcellulose, hydroxypropyl cellulose, hydroxypropyl methylcellulose (HPMC), hydroxyethyl cellulose, hydroxymethyl cellulose, sodium carboxymethylcellulose, carboxymethyl cellulose, nitrocellulose, methylcellulose or mixtures thereof.

7. The process according to claim 6, wherein rate-controlling polymer is hydroxypropyl methyl cellulose.

8. The process according to claim 7, wherein the amount of hydroxypropyl methyl cellulose is between 25.0% to 70.0% by weight of the total composition.

9. The process according to any one of the claims 1, wherein diluent is selected from the group comprising microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

10. The process according to claim 9, wherein diluent is microcrystalline cellulose.

11. The process according to claim 10, wherein diluent is microcrystalline cellulose and its average particle size D50 is 70-140 µm and loose density is 0.35-0.46 g/ml.

12. The process according to claim 11, wherein diluent is microcrystalline cellulose with code name PH 302.

13. The process according to any one of the claims 10 to 12, wherein the amount of microcrystalline cellulose is between 25.0% to 60.0% by weight of the total composition.

14. The process according to any one of the claims 1 to 5 , wherein lubricant is selected from the group comprising from magnesium stearate, calcium stearate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, sodium stearyl fumarate, sodium lauryl sulphate or mixtures thereof.

15. The process according to claim 1, the process comprising the steps of:
a) Weighing the fampridine and all excipients,
b) Mixing fampridine and glidant homogenously,
c) Adding diluent into the mixture and mixing homogenously,
d) Sieving the mixture,
e) Granulation with alcohol or water or alcohol/water mixture and followed by drying the granules and sieving them,
f) Adding rate-controlling polymer as the external phase and mixing,
g) Sieving lubricant and adding into the mixture and mixing,
h) Pressing the prepared mixture into tablets to ensure the tablet weight meets the specified requirements.
